# EUROPEAN PATENT SPECIFICATION

(11) **EP 1 195 178 B1**
(45) Date of publication and mention of the grant of the patent: **22.03.2006**
(21) Application number: 00830659.9
(22) Date of filing: 06.10.2000
(51) Int. Cl.: A61P 5/06, A61P 5/14, A61P 11/02, A61P 11/04, A61P 37/04, A61K 31/715, A61K 33/18

(54) **Immunotrophic preparation for treating adenotonsillary hypertrophy**
Immunotrophische Preparation zur Behandlung von tonsilläre Hypertrophie
Préparation immunotrophique pour le traitement de l'hypertrophie amygdalaire

(43) Date of publication of application: 10.04.2002
(73) Proprietor: D.M.G. Italia Srl, 00040 Pomezia RM (IT)
(72) Inventor: Mercuri, Luigi, 00181 Roma (IT)
(74) Representative: Sarpi, Maurizio

(56) References cited:
- EP-A- 0 705 542
- WO-A-00/27408
- WO-A-96/36327
- WO-A-99/01044
- WO-A-99/21567
- DATABASE WPI Week 199944 Derwent Publications Ltd., London, GB; AN 1999-522600 XP002165306 & JP 11 228387 A (TAISHO PHARM CO LTD), 24 August 1999 (1999-08-24)
- LEHRER J F: "Hypertrophic lingual tonsils and cough [letter]." WESTERN JOURNAL OF MEDICINE, (1986 FEB) 144 (2) 229. , XP000989472
- DATABASE WPI Week 198321 Derwent Publications Ltd., London, GB; AN 1983-51259K XP002165307 & SU 942 757 A (KIEV LARYNGOLOGY), 15 July 1982 (1982-07-15)
- DOI Y ET AL: "Free amino acids in human tonsillar tissue." CLINICAL CHEMISTRY, (1975 MAR) 21 (3) 414-7, XP000987109

## Description

The present invention relates to the dietary, pharmaceutical field and more particularly a preparation in the form of a syrup based on beta-glucan, arginine and iodine with the addition of B-group vitamins which is capable of fighting the adenotonsillary hypertrophy by improving the normal activity of the immunity system and helping the trophism of the lymphoid adenotonsillary tissue. The preparation is particulary suitable for children as the presence of L-arginine improves the condition of their growth by helping the production of the growth hormone which people having frequent night awakings are usually lacking of.

It is known that the symptoms of the simple substenosant adenotonsillary hypertrophy are a sometimes considerable decrease in the respiratory capacity as well as pathological catarrhal episodes. The result of such physiological troubles can be different but certainly disturbance of sleep and swallowing problems, among others, are the most troublesome ones. Additionally, as the growth hormone (G.H.) is mainly secreted during the steps 3-4 of the sleep, children suffering from simple adenotonsillary hypertrophy who do not have a regular sleep can show a reduced growth. The hypertrophic state of the mucous-lymphoid adenotonsillary tissue can be originated by several concomitant causes, however, two of them are the most important ones: the bad trophism of the mucous tissue and the immunity equilibrium against an invader that is hard to defeat. The ideal thing would be to operate with a treatment aiming at:
1) reducing the volume of the lymphoreticular mucous tissue with the result of improving the respiration and normalizing the sleep,
2) stimulating the reduced secretion of G.H., thus helping the growth,
3) supporting the patient physically,
4) reducing the frequency of the catarrhal episodes.

The present invention seeks to provide a preparation in the form of a syrup that allows the above-mentioned treatment to be carried out. According to the invention, a preparation is made in the form of a syrup, in which arginine, beta-glucan, iodine and B-group vitamins are included as proximate principle, by following the rational method described herebelow.

Arginine is an aminoacid which is widespread in the nature and mainly contained in the proteins of animal origin. It performs important functions in the cellular metabolism and operates particularly in the biosynthesis of urea in the Krebs cycle.

Arginine is not an essential aminoacid in itself but is considered as such in the period of growth and is indispensable for the optimum use of the aminoacid mixtures. (Chaitow L.: Aminoacids and their effects to our health, Tecniche Nuove 34-38, 1983).

In fact, arginine takes part in the synthesis of creatine and has an important anabolic action helping the metabolism. In addition, it regulates the release of the nitric oxide (NO) in the human body. The latter is an important endogenous messenger which regulates the vascular tone and the microcirculatory system and mainly is formed in the endothelial, neuronal cells as well as in the macrophages. For this reason it has a vasoregulating, neuro- and immunomodulating effect. These features, in case arginine is assumed in strong doses, are expressed by a regenerating activity of the tissues, particularly hepatic and muscular tissues, the healing of the injuries and the immunostimulating activity, the latter being connected in particular to the production of favouring NO which is, among others, the killer of intracellular macrophages (Green S.J., Meltzer M.S., Hibbs J.B. Jr., Nacy C.A., J. Immunol. 1990 Jan., 144: 1,278-283).

According to the above features, arginine is able to stimulate the synthesis of the growth hormone (STH). In fact, it has been used in the therapy of the hypopituitarism which delayes the somatic development (Kiseleva E.V., Zarubina N.A., Pankova S.S., Starkova N.T., Probl.Endokrinol.(Mosk) 1991 May; 37(3): 28-29). Glucans are natural polysaccharides consisting of glucopyranosic units and are found in nature in wheat, barley, oat, several microrganisms, and fungi.

Glucans generally derived from cereals are essential components of a diet, however, the purified beta-glucans, specifically beta-1-3-glucan, derived from yeast have been selectively identified as strong stimulating agents of the immunity system (Kokashis P.L., Williams D.L., Cook J.A., and Di Luzio N.R., Increased Resistance to Staphylococcus Aureus Infection And Enhancement In Serum Lysozyme Activity By Glucan. Science 199, 1340-1342 (1978)). They have the feature of activating macrophages, neutrophils and other cells having specific receptors for these compounds. The activation of such cells by beta-glucan stimulates the not specific immunity response by the organism. Beta-Glucan strengthens the defence mechanism of the organism under different conditions. It is proved that the cause therefor is the increased rate of phagocytosis through activated microphages (Sherwood E.R., Williams D.J., and Di Luzio N.R., Glucan stimulates production of antitumor cytolytic, cytostatic factors by macrophages. J. Biol. Resp. Mod-6, 358-381 (1986)).

It is also proved that the beta-glucans have specificity for the membrane complement receptor C3 (Ross G.D., Cain J.A., Myones B.L. et al. "Specificity of membrane complement receptor type three (CR3) for beta-glucans", Complement 4:61-74.1987).

In order to evaluate the immunity aspect, it should be remembered that our defence can develop through two ways:
a) the innate immunity that is a nonspecific immunity form and consists of gastric juices, skin, lysosomes, complement complex, macrophages, and neutrophils;
b) the acquired immunity that is a specific memory immunity form and consists of lymphocytes T and B.

In an attempt of fighting the invader, the reduced or delayed biological reaction of our immunity system produces an enhancement in the volume of the lymphoreticular tissue at the tonsilles that can generate a hyperplasia and cause the hypertrophy.

Modifying the macrophage response by its enhancement means to help the first line of defence of our organism by improving the reaction against "generic invaders". Stimulating the lymphocytic response means to activate the memory immunity against a "specific invader" and to start a defence directed exclusively against that invader and not others. Furthermore, no one can say that the activation of lymphocytes T occurs in T-Helper.

However, both systems of innate and acquired immunities operate in combination and are reciprocally activated up to the suppression of the "invader".

Factor C3 of the complement complex is doubtless the focusing point as it acts as a hinge between acquired immunity and innate immunity. In fact, it is responsible for the activation of macrophages and neutrophiles, and upon activation of macrophages it is responsible for helping the production of lymphokine Interleukin 1 that promotes the growth and the reproduction of lymphocytes capable of producing lymphokines from Interleukin 2 to Interleukin 6 as well as a factor that stimulates the colonies of granulocytes and monocytes and then neutrophiles and macrophages. It is self-evident that factor C3 of the complement complex is able to activate all of the innate and acquired immunity functions so that such factor is very important for the defence of the organism. Therefore, beta-glucan which is able to react with factors B and D of the complement complex and to activate factor C3 of such complex is also capable of helping the action of the whole immunity system.

Iodine performs an active function in the metabolism of the lymphatic glands and improves the trophism of the lymphoreticular tissue. Even if the iodine absorption mechanism and its action to the lymphoreticular tissue is unknown, one can suppose that alimentary iodine circulating in the form of iodide is captured by 90% by thyroid where upon oxidation it becomes metal iodine which will be released as T3-T4 to the extent of 1% daily and absorbed, for example, by the mammary glands, gastric mucose, and lymphoreticular tissue, thus taking part significantly in the trophism of such organes. Therefore, upon speaking of tonsillary lymphoreticular tissue one can infer that, in case of hypertrophy of the tonsillary tissue, an increase in the supply of iodine can cause a remarkable improvement in the trophism of such tissue and take a positive part in the normalization of the gland activity.

The B-group vitamins used are B5, B6, and B12.

The metabolic consequences of the lacking of vitamin B5 or pantothenic acid have been pointed out by P.C. Fry and his team since 1976. Vitamin B5 is used in otorhinolaryngology in case of chronic inflammation of the upper airways.

It is known that vitamin B6 or pyridoxine takes part in the immunity defense. In fact the lacking of vitamin B6 causes changes in the cell and humor immunities, i.e. decrease in the number of limphocytes, level of interleukin, and response to mitogens. Investigations are made to evaluate the effectivenness of an additional supply.

The therapeutic action of vitamin B12 in the anemias, particularly pernicious anemia, is based on a sound biochemical, clinical documentation. It is apparent that the supply thereof contributes to support the subject physically.

The following preferred formulation of the immunotrophic preparation in the form of syrup according to the present invention has been determined in the experiments carried out (Table I).

**TABLE I**

| | For 100 ml |
|---|---|
| Proteins | 5 g |
| Carbohydrates | 40.24 g |
| Fats | 0.9 g |
| Arginine | 5 g |
| Vitamin B5 | 60 mg |
| Vitamin B6 | 10 mg |
| Vitamin B12 | 10 mcg |
| Iodine | 2.000 mg |
| Beta-Glucan | 40 mg |
| Distilled water | q.s. |

The ranges within which the weight amounts of the proximate principles may vary under essentially unchanged effectiveness of the therapeutic action of the product are shown in the following Table II still with reference to 100 ml.

**TABLE II**

| | |
|---|---|
| Arginine | 4 ÷ 10 g |
| Vitamin B5 | 48 ÷ 120 mg |
| Vitamin B6 | 8 ÷ 20 mg |
| Vitamin B12 | 8 ÷ 20 mcg |
| Iodine | 1.600 ÷ 4.000 mg |
| Beta-Glucan | 32 ÷ 80 mg |

The addition of iodine is preferably provided by potassium iodide.

Further ingredients used can be dyes, preservatives and/or flavourings. By way of example, there may be mentioned: fructose, sodium benzoate, methylparaoxy benzoate as well as aromas such as pineapple and lemon.

Generally, the preparation of the present invention can be given to a patient in daily doses of 5 or 10 ml to stimulate the reaction of the immunity system. However, those skilled in the art can determine the length of the period during which the preparation is given and the doses thereof with respect to the general physical conditions of the patient and the importance of the hypertrophy to be treated.

Table III shows the nourishing information referred to one daily dose of 10 ml.

**TABLE III**

| Nourishing Information | Daily dose of 10 ml | R.D.A. (%) |
|---|---|---|
| Energy value | Kcal 18.9 | |
| | Kj 78.7 | |
| Proteins | 0.5 g | |
| Carbohydrates | 4.02 g | |
| Fats | 0.009 g | |
| Arginine | 0.5 g | |
| Vitamin B5 | 6 mg | 100% |
| Vitamin B6 | 1 mg | 50% |
| Vitamin B12 | 1 mcg | 100% |
| Iodine | 200 mcg | 133% |
| Glucan | 4 mg | |

In the light of the foregoing, once the adenotonsillary hypertrophy is diagnosed to a patient, the preparation of the invention allows as follows:
- giving him physical support and protection of the mucosa by vitamins B5, B6, B12 and L-arginine;
- improving the condition of his growth by helping the production of the growth hormone by L-arginine;
- helping the trophism of the adenotonsillary lymphoid tissue by iodine;
- improving the normal activity of the immunity system by beta-glucan.

## Claims

1. A preparation in the form of syrup to be used for the treatment of adenotonsillary hypertrophy, **characterized in that** it includes as proximate principles beta-glucan, arginine and iodine with the addition of B-group vitamins.

2. The preparation in the form of syrup of claim 1, **characterized in that** the proximate principles for 100 ml of product are included in the following ranges:
| | |
|---|---|
| Arginine | 4 ÷ 10 g |
| Vitamin B5 | 48 ÷ 120 mg |
| Vitamin B6 | 8 ÷ 20 mg |
| Vitamin B12 | 8 ÷ 20 mcg |
| Iodine | 1.600 ÷ 4.000 mg |
| Beta-Glucan | 32 ÷ 80 mg |

3. The preparation in the form of syrup of the preceding claims, **characterized in that** it includes the following composition referred to 100 ml of syrup:
| | For 100 ml |
|---|---|
| Proteins | 5 g |
| Carbohydrates | 40.24 g |
| Fats | 0.9 g |
| Arginine | 5 g |
| Vitamin B5 | 60 mg |
| Vitamin B6 | 10 mg |
| Vitamin B12 | 10 mcg |
| Iodine | 2.000 mg |
| Beta-Glucan | 40 mg |
| Distilled water | q.s. |

4. The preparation in the form of syrup of the preceding claims, **characterized in that** it is to be administered in daily doses of 5 or 10 ml.

## Patentansprüche

1. Zubereitung in Form eines Sirups zur Behandlung der adeno-tonsillaren Hypertrophie, **dadurch gekennzeichnet, dass** sie als unmittelbare Wirkstoffe Beta-Glucan, Arginin und Jod mit dem Zusatz von Vitaminen der B-Gruppe enthält.

2. Zubereitung in Form eines Sirups nach Anspruch 1, **dadurch gekennzeichnet, dass** die unmittelbaren Wirkstoffe für 100 ml des Produkts in den nachfolgenden Bereichen enthalten sind:
| | |
|---|---|
| Arginin | 4 ÷ 10 g |
| Vitamin B5 | 48 ÷ 120 mg |
| Vitamin B6 | 8 ÷ 20 mg |
| Vitamin B12 | 8 ÷ 20 µg |
| Jod | 1.600 ÷ 4.000 mg |
| Beta-Glucan | 32 ÷ 80 mg |

3. Zubereitung in Form eines Sirups nach einem der vorhergehenden Ansprüche, **dadurch gekennzeichnet, dass** sie die nachfolgende Zusammensetzung, bezogen auf 100 ml Sirup, enthält:
| | Pro 100 ml |
|---|---|
| Proteine | 5 g |
| Kohlenhydrate | 40,24 g |
| Fette | 0,9 |
| Arginin | 5 g |
| Vitamin B5 | 60 mg |
| Vitamin B6 | 10 mg |
| Vitamin B12 | 10 µg |
| Jod | 2.000 mg |
| Beta-Glucan | 40 mg |
| Destilliertes Wasser | q.s. |

4. Zubereitung in Form von Sirup nach einem der vorhergehenden Ansprüche, **dadurch gekennzeichnet, dass** sie täglich in Dosen von 5 oder 10 ml verabreicht wird.

## Revendications

1. Une préparation sous forme de sirop pour être utilisée pour le traitement de l'hypertrophie adénotonsillaire, **caractérisée du fait qu'**elle comprend comme principes proximaux le béta-glucane, l'arginine et le iode avec l'addition de vitamines du B-groupe.

2. La préparation sous forme de sirop de la revendication 1, **caractérisée du fait que** le principes proximaux pour 100 ml de produit sont compris dans les intervalles suivants:
| | |
|---|---|
| Arginine | 4 ÷ 10 g |
| Vitamine B5 | 48 ÷ 120 mg |
| Vitamine B6 | 8 ÷ 20 mg |
| Vitamine B12 | 8 ÷ 20 mcg |
| Iode | 1.600 ÷ 4.000 mg |
| Béta-Glucane | 32 ÷ 80 mg |

3. La préparation sous la forme de sirop des revendications précédentes, **caractérisée du fait qu'**elle comprend la composition suivante rapportée à 100 ml de sirop:
| | Pour 100 ml |
|---|---|
| Protéines | 5 g |
| Carbohydrates | 40,24 g |
| Lipides | 0,9 g |
| Arginine | 5 g |
| Vitamine B5 | 60 mg |
| Vitamine B6 | 10 mg |
| Vitamine B12 | 10 mcg |
| Iode | 2.000 mg |
| Béta-Glucane | 40 mg |
| Eau distillée | q.s. |

4. La préparation sous forme de sirop des revendications précédentes, **caractérisée du fait qu'**elle doit être administrée en doses de 5 ou 10 ml par jour.
